# FASCICULE DE BREVET EUROPEEN

(11) **EP 2 688 506 B1**
(45) Date de publication et mention de la délivrance du brevet: **06.12.2017**
(21) Numéro de dépôt: 12709344.1
(22) Date de dépôt: 21.03.2012
(51) Int. Cl.: A61C 3/04, A61C 8/00, A61B 50/22, A61B 50/30

(54) **EQUIPEMENT MÉCANISÉ DE CHIRURGIE COMPRENANT UN INSTRUMENT ET UN PORTE INSTRUMENT, CONDITIONNEMENT ET PORTE INSTRUMENT CORRESPONDANTS**
MECHANISIERTE CHIRURGISCHE AUSRÜSTUNG MIT EINEM INSTRUMENT UND EINEM INSTRUMENTENHALTER, ENTSPRECHENDE VERPACKUNG UND INSTRUMENTENHALTER
MECHANIZED SURGICAL EQUIPMENT COMPRISING AN INSTRUMENT AND AN INSTRUMENT HOLDER, CORRESPONDING PACKAGING AND INSTRUMENT HOLDER

(30) Priorité: 21.03.2011 FR 1152287
(43) Date de publication de la demande: 29.01.2014
(73) Titulaire: Neolix, 53001 Laval (FR)
(72) Inventeur: PERNOT, Jacques, 25870 Vieilley (FR); EUVRARD, Hubert, 25000 Besancon (FR); PAUL, Emilien, 39700 Ranchot (FR)
(74) Mandataire: Gicquel, Frédéric
(86) Numéro de dépôt international: PCT/EP2012/055028
(87) Numéro de publication internationale: WO 2012/126957

(56) Documents cités:
- EP-A1- 1 836 991
- WO-A1-88/05289
- DE-A1- 2 746 313
- DE-A1- 10 146 905

## Description

### 1) Domaine de l'invention

Le domaine de l'invention est celui de la conception et de la fabrication d'équipements de chirurgie. Plus précisément, l'invention concerne des équipements mécanisés de chirurgie, comprenant des instruments à usage mécanisé (instruments rotatifs, vibrants, giratoires...). L'invention concerne notamment les équipements comprenant des instruments chirurgicaux tels que les forets, les fraises, les lames, et, dans le domaine de la chirurgie dentaire (odontologie), les instruments endodontiques, les fraises dentaires, les tenons d'implants, les curettes de détartrage.

L'exemple traité, pour illustration, mais de manière non limitative, concerne les équipements de chirurgie dentaire. L'invention s'applique cependant également à d'autres équipements de chirurgie, comme par exemple à des équipements mécanisés pouvant être utilisés dans le domaine de la chirurgie orthopédique.

### 2) Art antérieur

Un équipement mécanisé de chirurgie est un équipement qui permet de transmettre un mouvement à un instrument chirurgical, qui peut par exemple être un mouvement rotatif, un mouvement vibratoire, ou un mouvement giratoire (c'est à dire un mouvement de rotation de l'instrument alternativement dans un sens et dans l'autre).

Les équipements mécanisés de chirurgie résultent généralement de l'assemblage de deux composants, à savoir :
- un instrument, constituant l'outil spécifique nécessaire à l'acte de chirurgie, et
- un porte instrument, comme par exemple une pièce à mains ou un contre-angle, équipé de moyens de réception, de retenue et éventuellement d'actionnement de l'instrument.

Classiquement, un chirurgien ou un dentiste doit utiliser plusieurs instruments, successivement, lors d'une intervention sur un patient.

Pour cette raison, le porte instrument est conçu pour être compatible avec plusieurs modèles d'instruments.

Le document de brevet publié sous le numéro EP 2123 233 ou le document DE 10146905 décrit un exemple de porte instrument facilitant la mise en place et la retenue d'un instrument de chirurgie dentaire. Selon la technique décrite, le porte instrument est doté de moyens de verrouillage/déverrouillage, qui se présentent sous la forme d'un bouton poussoir intégré, actionné à l'aide d'un doigt de la main par laquelle le dentiste tient le porte instrument. Un appui sur le bouton poussoir agit dans le porte instrument en desserrant les moyens de retenue de l'instrument (mâchoires, pinces, levier, verrou...), et permet donc de libérer l'instrument. A l'inverse, le relâchement du bouton poussoir sollicite les moyens de retenue de telle sorte qu'ils viennent serrer un instrument, préalablement inséré dans le porte instrument.

Ces moyens de verrouillage/déverrouillage peuvent présenter d'autres formes (tirette, poussette), induisant le même mode de retenue de l'instrument.

Dans la pratique, le praticien saisit un instrument entre le pouce et l'index d'une de ses mains, éventuellement gantée, ou à l'aide de pinces brucelles.

Puis, il introduit cet instrument dans le porte instrument, qu'il tient par son autre main, en pressant le bouton poussoir (ou en actionnant tout autre moyen de verrouillage/déverrouillage) à l'aide d'un doigt de cette autre main.

De même, le praticien doit appuyer sur le bouton poussoir, d'une main, pour libérer axialement l'instrument et l'extraire, de l'autre main.

Ainsi, l'instrument et le porte instrument mobilisent les deux mains du praticien, et ils exigent que le praticien exerce une pression d'un doigt et de surcroît qu'il la maintienne, tant que l'instrument n'est pas complètement sorti ou introduit.

On note que les exigences de montage sont plus importantes encore avec certains instruments, tels ceux dits de type 1 selon la norme NF EN ISO 1797, qui présentent un diamètre de 2,35 mm, une gorge et un méplat, et sont destinés à être connectés sur un contre-angle. En effet, le praticien doit dans ce cas orienter angulairement l'instrument dans le porte instrument, tout en exerçant une poussée continue sur le bouton poussoir, afin de faire coïncider le méplat de l'instrument, constituant une partie mâle, avec une partie femelle constituée par un orifice présent dans le porte instrument. Une fois la position adéquate trouvée, le praticien peut relâcher le bouton poussoir, et l'instrument est alors maintenu verrouillé sur le porte instrument, de manière axiale et en rotation. Le mouvement généré par d'éventuels moyens d'actionnement intégrés au porte instrument peut alors être transmis à l'instrument.

Par ailleurs, le porte instrument et l'instrument sont supposés stériles, avant d'être assemblés l'un avec l'autre, pour que l'équipement assemblé soit lui-même stérile au moment où il est introduit dans la bouche du patient.

Or, le mode de montage décrit précédemment implique un contact direct entre les doigts d'une main du praticien et l'instrument, d'une part, et un contact direct entre l'autre main du praticien et le porte instrument, au niveau du bouton poussoir et des parties alentour (telles que le corps de tête du contre-angle), d'autre part. Il engendre donc un important risque de contamination de l'équipement assemblé.

Cet inconvénient est d'autant plus important lorsque le méplat de l'instrument doit être recherché, par manipulation de l'instrument dans le porte instrument. En effet, ce mode opératoire engendre une difficulté ergonomique particulière, ainsi qu'une incertitude quant au bon verrouillage du système, qui incitent certains praticiens à ne pas mettre de gants pour gagner en sensibilité tactile. Le risque de contamination augmente donc significativement dans ce cas.

En outre, les gants ne représentent pas une garantie absolue d'asepsie car ils peuvent eux-mêmes être contaminés, percés... Le contact direct entre les mains du praticien et l'instrument et le porte instrument engendre donc, dans tous les cas, un important risque sanitaire pour le patient.

### 3) Objectifs de l'invention

L'invention a notamment pour objectif de pallier les inconvénients de l'art antérieur.

Plus précisément, l'invention a pour objectif de proposer un équipement mécanisé de chirurgie comprenant un instrument et un porte instrument, améliorant ses conditions d'utilisation en termes d'asepsie.

L'invention a également pour objectif de fournir un tel équipement mécanisé de chirurgie comprenant un instrument et un porte instrument, améliorant ses conditions d'utilisation au niveau ergonomique.

### 4) Exposé de l'invention

Ces objectifs, ainsi que d'autres qui apparaîtront par la suite, sont atteints grâce à l'invention comme décrit dans la revendication 1, qui a pour objet un équipement mécanisé de chirurgie comprenant :
- au moins un porte instrument comprenant des moyens de réception et de retenue d'un instrument de chirurgie à usage mécanisé, et
- au moins un instrument de chirurgie à usage mécanisé, destiné à être monté sur ledit porte instrument, ledit instrument comprenant une queue destinée à coopérer avec le porte instrument et une lame active.
Lesdits moyens de réception et de retenue sont mobiles entre :
- une position de déverrouillage, dans laquelle ils permettent l'introduction de ladite queue de l'instrument dans le porte instrument ou le retrait de ladite queue du porte instrument, et ;
- une position de verrouillage, dans laquelle ils empêchent le retrait de ladite queue du porte instrument ;
et l'équipement mécanisé de chirurgie comprend un étui dans lequel ladite lame active dudit instrument peut être logée de façon amovible, ledit étui présentant un élément de coopération destiné à coopérer avec lesdits moyens de réception et de retenue pour provoquer leur passage de ladite position de déverrouillage à la position de verrouillage, ou inversement.

De cette façon, le montage de l'instrument sur le porte instrument, ou son démontage, peuvent donc être réalisé sans qu'aucun contact ne soit nécessaire entre le praticien et l'instrument, dont la lame active est logée dans l'étui, et en limitant les contacts nécessaires au niveau de la tête du porte instrument.

La queue de l'instrument est placée, et peut être maintenu en position, dans le porte instrument, sans que le praticien n'ait à appuyer sur un organe d'actionnement de type bouton poussoir intégré sur le porte instrument.

Le verrouillage ou le déverrouillage de la position de cette queue de l'instrument dans le porte instrument, permettant respectivement l'utilisation de l'instrument ou son démontage du porte instrument, est obtenu par une simple action de l'étui sur les moyens de réception et de retenue du porte instrument. En effet, l'organe d'actionnement permettant le passage de la position de verrouillage à la position de déverrouillage des moyens de réception et de retenue est situé à proximité de l'emplacement destiné à recevoir la queue de l'instrument, afin de pouvoir être actionné par l'étui dans lequel est logé la lame active de cet instrument. De plus, l'étui présente lui-même une configuration adaptée pour réaliser cet actionnement.

Un équipement selon l'invention permet donc une utilisation dans de meilleures conditions d'ergonomie et d'asepsie que les techniques antérieures.

Par ailleurs, l'étui peut faciliter l'identification, visuelle ou informatique, de l'instrument qu'il contient, dont les dimensions sont faibles et qui peut parfois être confondu avec d'autres instruments très proches.

De façon avantageuse, lesdites positions de déverrouillage et de verrouillage des moyens de réception et de retenue sont des positions stables.

Ainsi, le praticien peut facilement introduire la queue de l'instrument dans le porte instrument ou la retirer du porte instrument, quand les moyens de réception et de retenue sont dans leur position stable de déverrouillage. Quand la queue d'un instrument est introduite dans le porte instrument, le praticien peut agir sur l'étui afin que son élément de coopération agisse sur les moyens de réception et de retenue pour provoquer leur passage vers la position de verrouillage, afin que la queue de l'instrument soit ferment retenue. Cette position de verrouillage est également stable, ce qui permet d'éviter un déverrouillage accidentel de la queue de l'instrument. Une nouvelle action de l'élément de coopération de l'étui sur les moyens de réception et de retenue permet de retourner vers la position stable de déverrouillage. Ainsi, ces moyens de réception et de retenue bistables, c'est à dire pouvant être placés dans deux positions distinctes stables, permettent une grande facilité d'utilisation de l'équipement mécanisé de chirurgie, dans le respect des conditions d'asepsie.

Selon un mode de réalisation préférentiel, lesdits moyens de réception et de retenue comprennent :
- un organe d'accueil permettant de recevoir et de maintenir ladite queue de l'instrument, ledit organe d'accueil étant mobile entre une position ouverte, autorisant l'introduction de ladite queue dans ledit organe d'accueil ou le retrait de ladite queue dudit organe d'accueil, et une position de retenue dans laquelle elle retient ladite queue dans ledit organe d'accueil ;
- un organe de verrouillage/déverrouillage, mobile entre ladite position de verrouillage dans laquelle il maintient ledit organe d'accueil dans ladite position de retenue, et ladite position de déverrouillage dans laquelle il ne maintient pas ledit organe d'accueil dans ladite position de retenue ;
et en ce que ledit élément de coopération dudit étui est conformé pour coopérer avec ledit organe de verrouillage/déverrouillage pour provoquer son passage de ladite position de verrouillage à ladite position de déverrouillage, ou inversement.

Ainsi, par une simple action sur l'organe de verrouillage/déverrouillage, celui-ci peut alterner entre une position de verrouillage, dans laquelle l'instrument est fermement retenu dans l'organe d'accueil, et une position de déverrouillage, dans laquelle la mobilité de l'organe d'accueil permet le montage et le démontage de la queue d'un instrument.

En outre, lorsque l'organe de verrouillage/déverrouillage est dans sa position de déverrouillage, l'organe d'accueil peut être dans une position de retenue, permettant qu'un instrument soit retenu dans le porte instrument, mais de façon non verrouillée, de sorte qu'un effort manuel suffit à extraire l'instrument du porte instrument en faisant passer l'organe d'accueil de sa position de retenue à sa position ouverte.

Il est à noter que, de préférence, l'organe d'accueil est stable dans sa position de retenue, et instable dans sa position ouverte. Ainsi, il reste par défaut dans sa position de retenue, tant qu'un effort généré par l'introduction de la queue d'un instrument ou le retrait de cette queue ne l'entraîne pas en position ouverte.

La force de retenue de l'instrument peut donc être étagée, en fonction de la position (verrouillage/déverrouillage) de l'organe de verrouillage/déverrouillage, entre un maintien ferme permettant l'utilisation de l'instrument et un maintient faible permettant le retrait de l'instrument. Cet étagement contribue à améliorer l'ergonomie d'utilisation de l'équipement, car il permet d'éviter que l'instrument ne tombe du porte instrument quand les moyens de réception et de retenue sont en position de déverrouillage. Il facilite ainsi le montage et le démontage de l'instrument.

L'organe d'accueil peut présenter une forme intérieure complémentaire de la forme extérieure d'une grande variété d'instruments, lui permettant d'être compatible et efficace notamment avec un grand nombre d'instruments présentant un méplat.

Lors des opérations de maintenance, ou de changement du type d'instrument installé, l'organe d'accueil peut être facilement remplacé, sans nécessiter le remplacement d'autres composants du porte instrument. La forme extérieure de l'organe d'accueil peut pour cela être conçue suivant un standard, permettant à l'organe de verrouillage/déverrouillage d'être compatible avec différents modèles d'organe d'accueil.

Selon une solution avantageuse, ledit porte instrument comprend un corps dans lequel l'organe de verrouillage/déverrouillage est monté coulissant, ledit organe de verrouillage/déverrouillage présentant au moins un ergot mobile destiné à coopérer avec au moins un logement ménagé dans ledit corps.

Une action de l'étui peut ainsi facilement activer le coulissement de l'organe de verrouillage/déverrouillage, dans le but de provoquer le passage de l'organe de verrouillage/déverrouillage entre ses positions de verrouillage et de déverrouillage.

Le praticien a alors seulement besoin d'exercer une pression de l'étui contre l'organe de verrouillage/déverrouillage, pour permettre à l'organe d'accueil de passer librement à sa position ouverte permettant de sortir, ou introduire un instrument, ou au contraire pour permettre à cet organe d'accueil de maintenir fermement cet instrument.

La coopération d'un ergot avec un logement permet en outre de maintenir stable une ou plusieurs positions de l'organe de verrouillage/déverrouillage dans le corps. Ainsi, cette position de l'organe de verrouillage/déverrouillage est maintenue même si le praticien relâche la pression sur l'étui, ce qui constitue un avantage lorsqu'il doit introduire et positionner en rotation l'instrument dans le porte instrument.

L'alternance entre plusieurs positions stables de l'organe de verrouillage/déverrouillage dans le corps est réalisée grâce à la mobilité de l'ergot, déclenchée par le mouvement de coulissement de l'organe de verrouillage/déverrouillage.

De façon avantageuse, ledit ergot est mobile radialement et latéralement.

La mobilité à la fois radiale et latérale de l'ergot permet en effet, avec une forme adaptée de l'ergot et du logement, d'imposer l'ordre des positions que l'organe de verrouillage/déverrouillage peut successivement occuper dans le corps, afin par exemple de court-circuiter certaines positions d'arrêt. Ainsi, le montage et le démontage de l'instrument peuvent être réalisés en actionnant l'organe de verrouillage/déverrouillage, par l'intermédiaire de l'étui, suivant toujours le même sens de coulissement.

Par exemple, le coulissement réalisé par pression de l'étui sur l'organe de verrouillage/déverrouillage peut permettre le passage d'une première position, stable, où l'instrument est retenu et verrouillé dans le porte instrument, à une deuxième position, stable, où l'instrument est retenu de façon non verrouillée dans le porte instrument ; en prolongeant ce coulissement, toujours dans le même sens, une position non stable où l'instrument est toujours retenu de façon non verrouillée peut être atteinte, qui permet de revenir à la première position, par l'intermédiaire de moyens de rappel, sans que l'ergot puisse s'arrêter dans la deuxième position.

Autrement dit, l'ergot est mobile suivant des directions qui lui permettent de suivre un chemin différent lors du passage de la position de verrouillage à la position de déverrouillage et lors du passage de la position de déverrouillage à la position de verrouillage, ce qui permet de réaliser ces deux passages en conservant le même sens de coulissement et donc en exerçant le même actionnement de l'organe de verrouillage/déverrouillage.

Ainsi, le praticien peut monter et démonter ses instruments du porte instrument par une action simple et intuitive. L'interface d'actionnement étant constituée uniquement d'un étui, dont l'action est concentrée sur l'organe de verrouillage/déverrouillage, l'amélioration de l'ergonomie s'accompagne d'une amélioration des conditions aseptiques d'utilisation de l'équipement.

On note que le corps évoqué, compris dans le porte instrument et dans lequel est ménagé le logement, peut être :
- fixe par rapport au bâti du porte instrument (auquel cas il peut même faire partie de ce bâti) ;
- mobile par rapport au bâti du porte instrument, notamment lorsqu'il participe à l'entraînement du mouvement de l'instrument (en rotation, en particulier).

De façon préférentielle, ledit ergot est élastiquement déformable.

Ainsi, il peut être amené sans effort ni frottement excessifs dans plusieurs positions, relativement au corps du porte instrument, et notamment une position où il pénètre le logement, avec une force qui à la fois permet d'assurer la stabilité de la position de l'organe de verrouillage/déverrouillage par rapport au corps, et peut être facilement vaincue lorsque le praticien veut, par l'intermédiaire de l'étui, provoquer le coulissement de l'organe de verrouillage/déverrouillage.

Un tel ergot peut comporter un ou plusieurs éléments mécaniques avec un système de ressort. Cependant, il sera de préférence réalisé dans un matériau souple, plastique ou métallique, au même titre d'ailleurs que l'ensemble de l'organe de verrouillage/déverrouillage.

Avantageusement, ledit organe de verrouillage/déverrouillage est monté coulissant par rapport audit organe d'accueil.

Ce mouvement de coulissement peut alors être exploité pour amener l'organe de verrouillage/déverrouillage dans un premier temps dans la position de déverrouillage , dans laquelle l'organe d'accueil peut passer de sa position de retenue à sa position ouverte position ouverte, autorisant l'introduction de l'instrument dans le porte instrument ou le retrait de l'instrument à partir du porte instrument, puis dans un second temps dans la position de verrouillage, dans laquelle il maintient l'organe d'accueil dans sa position de retenue de l'instrument dans le porte instrument.

L'organe de verrouillage/déverrouillage étant monté coulissant à la fois par rapport au corps du porte instrument et par rapport à l'organe d'accueil, ce dernier peut avantageusement être fixe par rapport au corps du porte instrument.

Le mouvement de coulissement est alors unique et commun, il ne concerne que l'organe de verrouillage/déverrouillage, et l'assemblage de ce dernier avec le corps et l'organe d'accueil peut être conçu d'autant plus compact, solide et efficace pour retenir l'instrument.

Afin de convertir ce mouvement de coulissement en un maintien de l'organe d'accueil dans sa position de retenue de l'instrument, des formes complémentaires (coniques, par exemple) peuvent être adaptées sur l'organe d'accueil et sur l'organe de verrouillage/déverrouillage.

Selon une solution préférentielle, ledit organe d'accueil présente une base pourvue d'au moins un doigt mobile radialement, ladite queue de l'instrument présentant au moins un évidement destiné à recevoir ledit doigt dans ladite position de retenue.

De cette façon, dans la position de retenue de l'organe d'accueil, l'instrument est retenu axialement dans l'organe d'accueil grâce à l'application d'une force radiale sur au moins un doigt, qui peut prendre la forme d'un point, d'une arête ou de tout autre ensemble de points, continu ou discontinu, la force radiale permettant à ce doigt de prendre place dans un évidement de l'instrument, tel qu'une gorge, pour empêcher son retrait ou, tout au moins, pour imposer qu'un effort manuel minimal soit exercé pour le retirer.

Chaque doigt de l'organe d'accueil peut occuper une position, dans la position ouverte de l'organe d'accueil, qui est plus éloignée radialement que lorsqu'il retient un instrument, quand l'organe d'accueil est en position de retenue. Cette position ouverte, permettant l'introduction et le retrait d'un instrument, est avantageusement une position instable.

Le doigt mobile radialement est maintenu de façon verrouillée dans la position permettant de retenir l'instrument grâce au coulissement évoqué précédemment de l'organe de verrouillage déverrouillage vers sa position de verrouillage, dans laquelle il exerce un resserrement de l'organe d'accueil sur l'instrument empêchant le mouvement des doigts vers la position ouverte.

Ainsi, un instrument destiné à être introduit dans un porte instrument peut être :
- orienté en rotation dans l'organe d'accueil, grâce à la présence d'un ou de plusieurs plats ou méplats sur la queue de l'instrument, coopérant avec l'intérieur de l'organe d'accueil ;
- retenu axialement dans l'organe d'accueil, grâce à la présence d'un ou de plusieurs doigts, mobiles radialement à la base de l'organe d'accueil et coopérant au niveau d'évidements dans l'instrument ;
- retenu de façon verrouillée, à l'intérieur de l'organe d'accueil, par contact entre les doigts et un évidemment de la queue de l'instrument, voire par contact de l'intérieur de l'organe d'accueil avec les plats ou méplats de la queue de l'instrument.

On note que l'élasticité de l'organe d'accueil peut permettre de supprimer le jeu entre le porte instrument et l'instrument, améliorant de ce fait leur coopération (réduction des vibrations) ainsi que le ressenti tactile du praticien (sensation plus fine).

De façon avantageuse, ledit porte instrument comporte un ressort de rappel entre ledit corps et ledit organe de verrouillage/déverrouillage, ledit ressort tendant à pousser ledit organe de verrouillage/déverrouillage vers ladite position de verrouillage.

Lors du montage coulissant de l'organe de verrouillage/déverrouillage dans le corps, ce ressort peut ainsi être installé en position comprimée entre ces deux éléments, de telle sorte qu'il exerce une poussée continuelle sur l'organe de verrouillage/déverrouillage, en direction d'une position de verrouillage.

Une pression de l'étui sur l'organe de verrouillage/déverrouillage, dans le sens contraire à la pression exercée par le ressort sur l'organe de verrouillage/déverrouillage, permet alors de faire coulisser ce dernier jusqu'à une position de déverrouillage, dans laquelle l'organe d'accueil peut passer dans sa position ouverte pour autoriser l'introduction de l'instrument dans le porte instrument ou le retrait de l'instrument à partir du porte instrument.

Le ressort contribue à stabiliser les positions de verrouillage et de déverrouillage de l'organe de verrouillage/déverrouillage, éventuellement en complément de la coopération de l'ergot avec un logement dans le corps, en supprimant les jeux susceptibles d'être présents dans ces positions.

Ainsi, le praticien profite non seulement du fait de pouvoir relâcher la pression exercée par l'étui sur l'organe de verrouillage/déverrouillage, mais il gagne aussi en précision et en sensibilité tactile, lorsqu'il introduit l'instrument dans le porte instrument.

Ensuite, une nouvelle compression puis un relâchement du ressort permettent à l'organe de verrouillage/déverrouillage de quitter la position de déverrouillage et de prendre la position de verrouillage, dans laquelle il maintient l'organe d'accueil en position de retenue de l'instrument.

Le ressort constitue donc un moyen de rappel, simple à mettre en oeuvre avec le coulissement de l'organe de verrouillage/déverrouillage dans le corps et qui contribue à l'amener dans différentes positions stables, dans lesquelles le praticien peut facilement utiliser, monter ou démonter l'instrument.

L'ergonomie d'utilisation et par conséquent les conditions d'asepsie sont améliorées, car le praticien n'a besoin ni de toucher l'instrument (il agit sur l'étui, en tenant le porte instrument), ni d'exercer une pression continue sur l'organe de verrouillage/déverrouillage, pour installer l'instrument.

Lors de l'introduction de l'instrument, la solidité du maintien ferme de l'instrument sur le porte instrument se trouve naturellement vérifiée, sans geste supplémentaire du praticien, du fait même que l'instrument reste retenu sur le porte instrument et non plus enserré dans l'étui pincé par le praticien.

Avantageusement, ledit organe de verrouillage/déverrouillage présente une partie s'étendant hors du corps du porte instrument.

De cette façon, l'organe de verrouillage/déverrouillage reste visible et facilement accessible pour le praticien, qui doit agir sur cet organe de verrouillage/déverrouillage, par l'intermédiaire de l'étui, lorsqu'il souhaite installer ou démonter un instrument.

Cela signifie également que l'organe de verrouillage/déverrouillage, mobile par rapport au corps du porte instrument, ne rentre pas complètement dans le volume du corps sans une intervention extérieure, telle qu'une pression de l'étui. Le ressort de rappel, notamment, peut alors contribuer à ce que l'organe de verrouillage/déverrouillage présente une partie s'étendant par défaut hors du corps du porte instrument.

De façon préférée, ladite partie est cylindrique.

Cette forme est favorable à une répartition homogène des efforts transmis par l'intermédiaire de l'étui, avec lequel la partie est susceptible de coopérer lorsque le praticien souhaite installer ou démonter un instrument, en particulier dans le cas où l'élément de coopération de l'étui est une collerette.

Par ailleurs, cette forme cylindrique peut permettre de combler des jeux et évidements présents entre le corps du porte instrument et l'organe d'accueil, et limiter ainsi le risque d'encrassement, voire de coincements, lorsque l'organe de verrouillage/déverrouillage est en mouvement.

Préférentiellement, ledit élément de coopération de l'étui est une collerette. Ainsi, l'effort exercé sur l'étui peut être uniformément réparti, à la surface d'une pièce en forme d'anneau centré sur l'instrument, lors des opérations de montage et de démontage qui amènent l'étui à coopérer avec l'organe de verrouillage/déverrouillage.

### 5) Liste des figures

D'autres caractéristiques et avantages de l'invention apparaîtront plus clairement à la lecture de la description suivante de modes de réalisation de l'invention, donnés à titre d'exemples illustratifs et non limitatifs, et des dessins annexés, parmi lesquels :
- la figure 1 illustre partiellement, selon une vue en perspective, un ensemble composé d'un porte instrument et d'un instrument de chirurgie dentaire selon un mode de réalisation de l'invention ;
- la figure 2 illustre partiellement, selon une vue en perspective, un instrument selon un mode de réalisation de l'invention ;
- les figures 3a, 3b et 3c illustrent partiellement, en coupe, un instrument selon trois modes de réalisation préférentiels de l'invention ;
- la figure 4 illustre, en vue de face, un instrument, un étui et un moyen d'emballage selon un mode de réalisation de l'invention ;
- les figures 5a et 5b illustrent partiellement, en coupe, l'assemblage d'un instrument dans un étui réalisé selon deux modes de réalisation préférentiels de l'invention ;
- la figure 6 illustre, en perspective, le porte instrument de la figure 1 (partiellement), et l'instrument, l'étui et le moyen d'emballage de la figure 4, le moyen d'emballage partiellement ouvert contenant l'étui et, à l'intérieur de l'étui, l'instrument ;
- la figure 7 illustre, en vue de côté, le porte instrument (partiellement), l'instrument et l'étui de la figure 6, dans une position de montage ;
- la figure 8 illustre, en coupe, un équipement pour dentiste selon un mode de réalisation de l'invention, avec l'organe d'accueil dans une position de retenue et l'organe de verrouillage/déverrouillage dans une position de déverrouillage ;
- la figure 9 illustre, en vue de face, le corps de porte instrument de l'équipement de la figure 8, dans une position correspondant à celle de la figure 8 ;
- la figure 10 illustre, en coupe, l'équipement pour dentiste de la figure 8, avec l'organe d'accueil dans une position de retenue et l'organe de verrouillage/déverrouillage en transition entre ses positions de déverrouillage et de verrouillage ;
- la figure 11 illustre, en vue de face, le corps de porte instrument de l'équipement de la figure 10, dans une position correspondant à celle de la figure 10 ;
- la figure 12 illustre, en coupe, l'équipement pour dentiste des figures 8 et 10, avec l'organe d'accueil dans une position de retenue et l'organe de verrouillage/déverrouillage dans une position de verrouillage ;
- la figure 13 illustre, en vue de face, le corps de porte instrument de l'équipement de la figure 12, dans une position correspondant à celle de la figure 12 ;
- les figures 14a et 14b illustrent, selon une vue en perspective, un organe d'accueil réalisé selon deux modes de réalisation préférentiels de l'invention ;
- la figure 15 illustre, selon une vue en perspective, un porte instrument, un instrument, un étui selon un mode de réalisation de l'invention, ainsi qu'un moyen de stockage temporaire dans lequel repose l'étui contenant l'instrument.

### 6) Exposé de modes de réalisation de l'invention

### 6.1. Equipement mécanisé de chirurgie

Tel qu'indiqué précédemment, le principe de l'invention réside dans le fait de réaliser un équipement mécanisé de chirurgie comprenant un porte instrument et un instrument, destinés à être assemblés et désassemblés sans que le praticien n'ait à toucher ni l'instrument ni le corps de tête du porte instrument.

Les modes de réalisation qui sont décrits ci-après, en lien avec les figures, portent sur un tel équipement mécanisé de chirurgie destiné à la chirurgie dentaire. L'invention ne se limite cependant pas à ces applications possibles au domaine de la chirurgie dentaire. L'homme du métier peut ainsi sans difficulté la mettre en oeuvre dans d'autres domaines de la chirurgie mettant en oeuvre des équipements de chirurgie à usage mécanisé, comme par exemple le domaine de la chirurgie orthopédique, dans lequel des instruments à usage mécanisé, comme par exemple des forets, sont couramment utilisés.

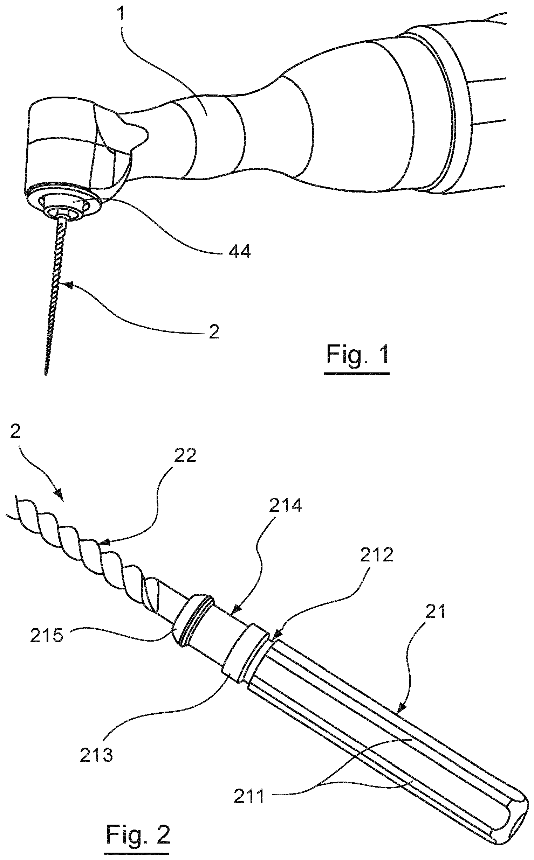

En référence à la figure 1, un équipement pour dentiste comprend deux composants, solidarisés l'un à l'autre lors d'une intervention chirurgicale :
- un porte instrument 1, en l'occurrence une tête de contreangle, formant un organe de maintien d'un instrument et
- un instrument chirurgical 2, ou foret, formant la partie active de l'équipement, monté dans le porte instrument.

### 6.2. Instrument de chirurgie

Selon la figure 2, l'instrument 2, de forme générale cylindrique, comprend une queue 21, destinée à coopérer avec le porte instrument, et une lame active 22, destinée à entrer dans la bouche du patient opéré par le dentiste. L'instrument doit donc, en particulier au niveau de la lame active 22, rester stérile.

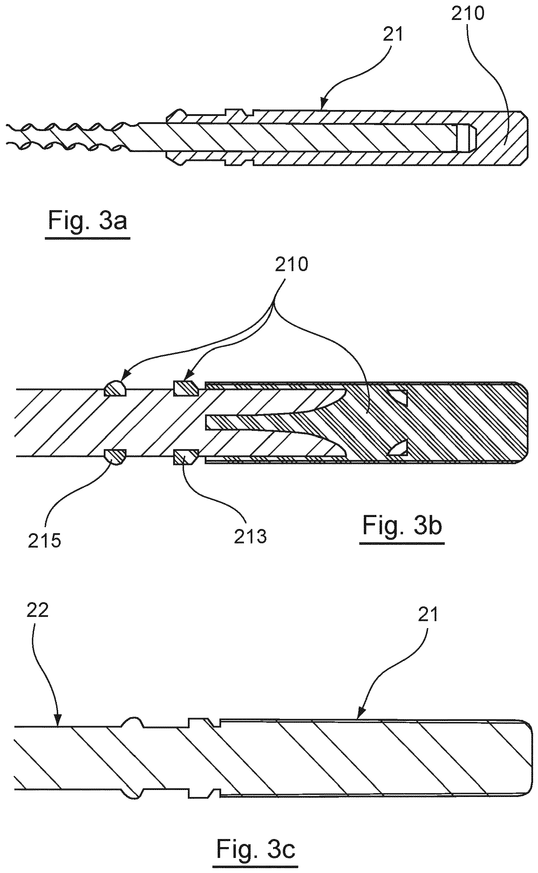

L'instrument 2 est, de préférence, réalisé par surmoulage plastique (cas illustrés sur les figures 3a et 3b) ou d'un seul tenant (figure 3c).

L'instrument 2 peut également être réalisé avec une queue 21 métallique, plastique, montée serrée sur la lame 22.

Le surmoulage est réalisé en une seule partie 210 (figure 3a), constituant alors la queue 21, ou en plusieurs parties 210 (figure 3b).

La lame active 22 est, dans tous les cas, réalisée dans un matériau métallique.

Dans le cas illustré sur la figure 2, la queue 21 comporte des plats 211 destinés à assurer un maintien en rotation de l'instrument 2.

Dans sa partie la plus proche de la lame active 22, la queue 21 comporte également deux gorges 212, 214, séparées par une collerette d'arrêt 213 incluant un épaulement. Une collerette biseautée 215 (qui pourrait également être de forme sensiblement arrondie, telle que sur un bossage) marque la jonction entre la gorge 214 et la lame active 22.

On note que, sur l'exemple illustré par la figure 3b, les collerettes d'arrêt 213 et biseautée 215 constituent des parties 210 surmoulées de façon dissociée.

### 6.3. Ensemble de l'instrument et de l'étui

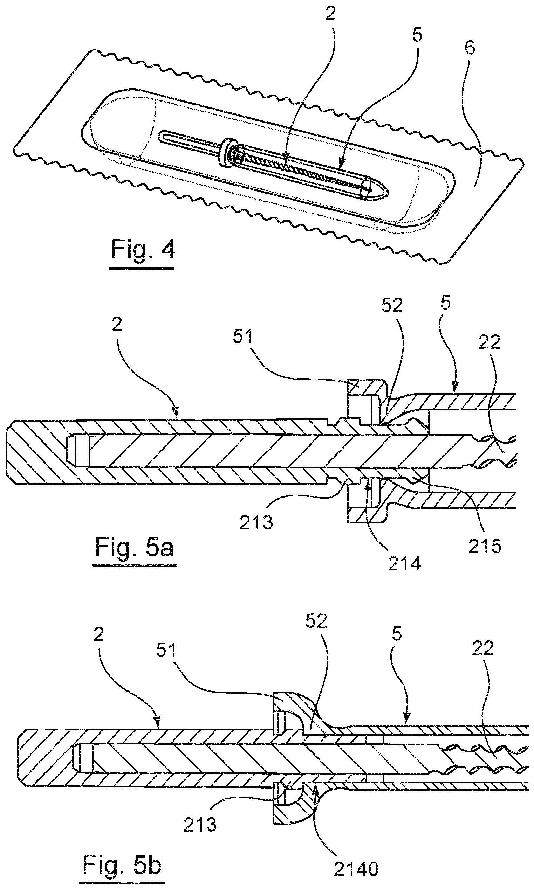

Les figures 4 à 7 présentent un ensemble constitué par l'instrument 2 et un étui 5, alors que la lame active 22 est logée dans un étui 5. Cet étui fait partie intégrante du conditionnement de l'instrument. Il permet de transporter et de manipuler ce dernier, sans toucher sa lame active 22.

Les figures 5a et 5b illustrent deux modes de réalisation préférentiels d'un ensemble constitué d'un instrument 2 et d'un étui 5.

La figure 5a illustre un instrument 2 surmoulé ou avec une queue métallique ou plastique rapportée (tel que sur la figure 3a) dont la lame active est dans un étui 5 semi-rigide, réalisé par exemple dans un matériau plastique. L'étui présente au moins une lèvre intérieure 52, souple, destinée à coulisser le long de la gorge 214 de l'instrument. La collerette d'arrêt 213 constitue une butée, destinée notamment à empêcher la chute de l'instrument au fond de l'étui (ce qui risquerait d'endommager la lame active 22), tandis que la collerette biseautée 215 constitue un obstacle au coulissement, qu'un simple effort manuel permet de vaincre, pour extraire l'instrument de l'étui ou l'y introduire.

Dans l'exemple illustré, la lèvre intérieure est unique, de forme annulaire, et elle coopère avec la gorge 214 au niveau d'un diamètre de cette dernière.

La figure 5b illustre un autre instrument 2, différant par le fait qu'il ne présente pas de collerette biseautée telle que dans l'exemple précédent, dans un autre étui 5, souple, réalisé dans un matériau tel que le silicone. L'étui présente une lèvre intérieure 52 allongée, sous la forme d'une enveloppe cylindrique, destinée à coulisser le long de l'extrémité cylindrique lisse 2140 de l'instrument. L'étui est représenté en butée contre la collerette d'arrêt 213 de l'instrument. Dans ce cas, ce sont les frottements entre la lèvre intérieure et l'extrémité cylindrique lisse 2140 qui constituent un obstacle au coulissement, qu'un simple effort manuel permet de vaincre pour extraire l'instrument de l'étui ou l'y introduire.

On note que, dans tous les cas, la collerette 51 de l'étui présente une rigidité suffisante pour supporter un effort manuel de poussée longitudinale.

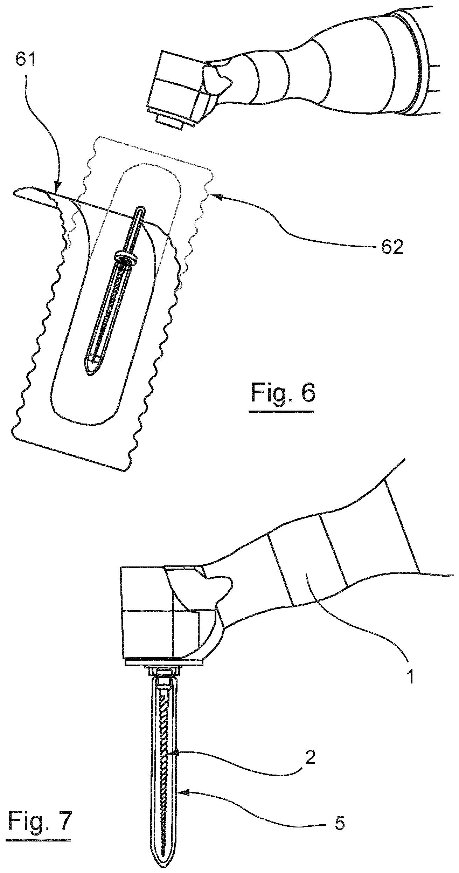

Afin d'améliorer encore les conditions d'asepsie dans lesquelles l'équipement peut être utilisé par le dentiste, l'étui contenant l'instrument est emballé, livré, apprêté dans un moyen d'emballage 6. Ce moyen d'emballage, jetable, réalisé dans un plastique souple, comprend deux parties 61, 62 dotées de parois minces et que le praticien sépare lorsqu'il veut utiliser l'instrument, protégé par l'étui contenu à l'intérieur (figure 6). Après ouverture partielle de l'emballage, l'instrument peut être approché du porte instrument, puis installé sur ce dernier, sans qu'aucun contact direct entre le praticien et l'instrument, voire entre le praticien et l'étui, ne soit nécessaire.

### 6.4. Moyens de réception et de retenue de l'instrument

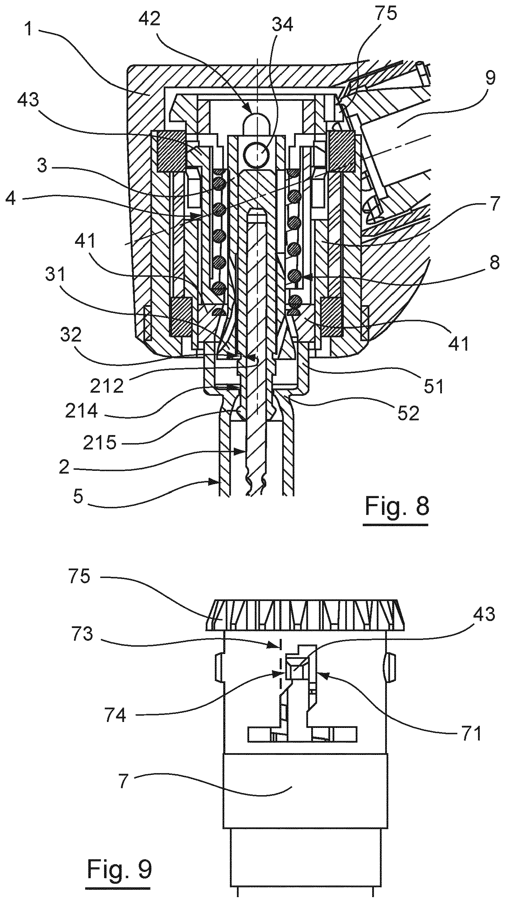

L'instrument 2 est reçu et retenu dans le porte instrument 1 par des moyen de réception et de retenue constitués d'un organe d'accueil 3 et d'un organe de verrouillage/déverrouillage 4, tels que représentés sur les figures 8, 10 et 12.

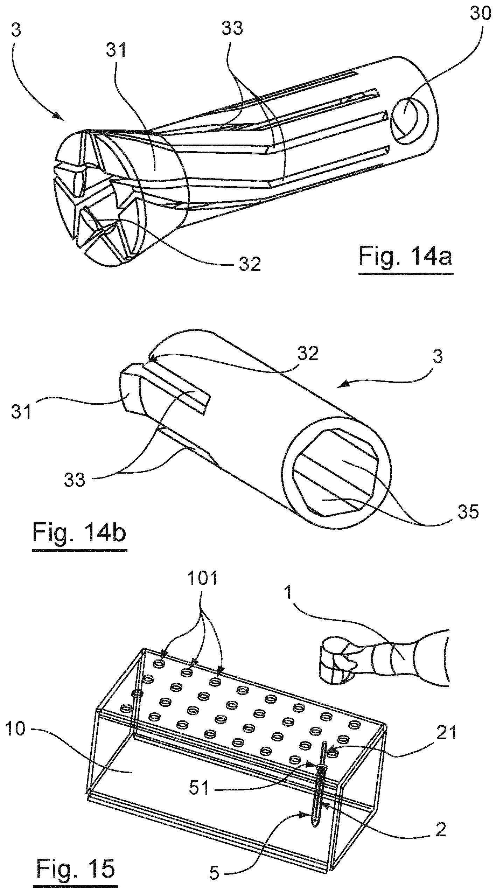

L'organe d'accueil 3, illustré de façon isolée sur les figures 14a et 14b, selon deux modes de réalisation préférentiels, présente une forme tubulaire.

A l'une de ses extrémités, il comporte un alésage diamétral 30 (non visible sur la figure 14b) destiné à recevoir un moyen de fixation, tel qu'un axe 34. A l'extrémité opposée, il présente une base 31 évasée suivant une forme conique et dotée d'une lèvre intérieure 32, saillante, qui s'étend radialement vers l'intérieur de l'organe d'accueil.

L'organe d'accueil comprend en outre des évidements longitudinaux 33 tels, qu'il peut se déformer de façon élastique, selon une direction radiale, principalement au niveau de sa base 31. Cette mobilité permet à la lèvre intérieure (ou doigt) 32 de rentrer dans la gorge 212 d'un instrument, en cours de montage, ou de se retirer de cette gorge.

Ainsi, l'organe d'accueil 3 présente une base 31 pourvue d'au moins un doigt 32 mobile radialement, et l'instrument 2 présente au moins un évidement 212 destiné à recevoir le doigt dans la position de retenue.

La déformation de l'organe d'accueil 3 lui permet d'osciller entre une configuration où il est suffisamment ouvert pour permettre l'introduction ou le retrait d'un instrument (position ouverte), et une configuration où il peut retenir un instrument, notamment par sa lèvre intérieure (ou doigt) 32 (position de retenue). De préférence, cet organe d'accueil présente une élasticité tendant à le ramener dans sa position de retenue, ou dans une position proche de sa position de retenue.

Par ailleurs, la partie intérieure creuse de l'organe d'accueil 3 présente des formes 35 (plats...) complémentaires des plats 211 de la queue 21 de l'instrument. Ainsi, l'instrument 2 introduit dans l'organe d'accueil occupe une position verrouillée en rotation.

L'organe de verrouillage/déverrouillage 4 présente une forme générale tubulaire et des dimensions qui lui permettent de contenir l'organe d'accueil 3.

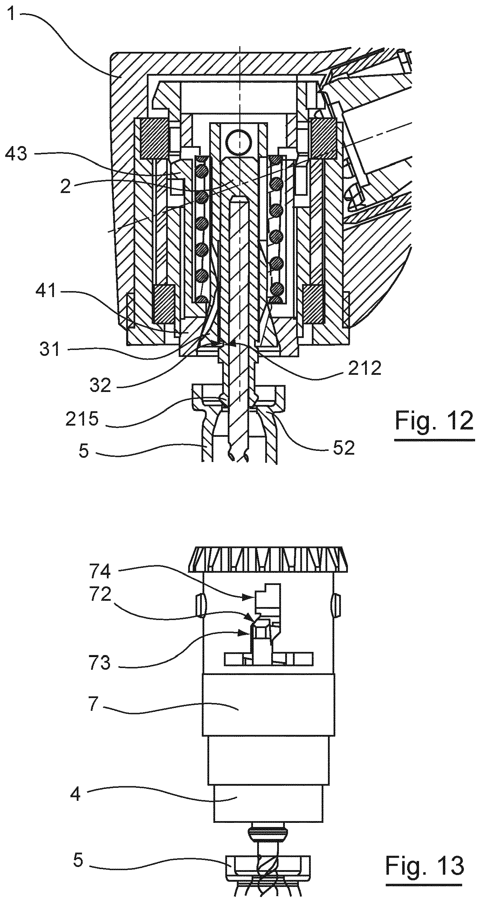

La base 41 de l'organe de verrouillage/déverrouillage 4 présente une forme intérieure conique, qui coopère avec la base conique 31 de l'organe d'accueil 3 (figure 12).

Lorsque la base 41 enveloppe la base 31, cette-dernière est contractée radialement, ce qui maintient l'organe d'accueil 3 dans sa position de retenue dans laquelle il retiens un instrument 2 préalablement introduit dans l'organe d'accueil 3. Inversement, l'éloignement de la surface conique de la base 31 par rapport à la surface conique de la base 41 relâche radialement l'organe d'accueil ce qui lui permet de passer dans une position ouverte où un instrument peut être introduit ou retiré.

Lors du montage et du démontage de l'instrument 2, l'étui 5 est amené contre le porte instrument 1 (figure 7). La surface extérieure de la collerette 51 de l'étui est alors en contact avec la surface extérieure de la base 41 de l'organe de verrouillage/déverrouillage (figure 8).

Il est à noter que l'instrument 2 possède un certain degré de liberté en rotation dans l'étui 5, en particulier dans le cas illustré par la figure 5a (lèvre intérieure 52 'annulaire'). L'instrument peut alors facilement tourner autour de son axe, dans l'étui, lorsqu'il est introduit dans l'organe d'accueil 3, afin d'atteindre une position dans laquelle ses plats 211 s'engagent contre les formes complémentaires 35 présentes à l'intérieur de l'organe d'accueil. Cependant, le praticien conserve toujours la possibilité de faire tourner entre ses doigts l'ensemble constitué de l'étui et de l'instrument, afin de faciliter l'introduction de ce dernier dans l'organe d'accueil.

Ainsi, l'équipement pour dentiste illustré comprend :
- au moins un porte instrument 1 comprenant des moyens de réception et de retenue d'un instrument 2 de chirurgie dentaire et
- au moins un instrument 2 de chirurgie dentaire destiné à être monté sur le porte instrument 1.

Plus précisément, les moyens de réception et de retenue comprennent :
- un organe d'accueil 3 de l'instrument, mobile entre une position ouverte, autorisant l'introduction de l'instrument dans le porte instrument ou le retrait de l'instrument à partir du porte instrument, et une position de retenue ;
- un organe de verrouillage/déverrouillage 4 de l'organe d'accueil en position de retenue.

En outre, l'équipement pour dentiste comprend un étui 5 dans lequel est logé l'instrument 2, cet étui présentant un élément de coopération, en l'occurrence une collerette 51, destiné à coopérer avec l'organe de verrouillage/déverrouillage 4 pour provoquer son passage de la position de verrouillage dans laquelle il verrouille l'organe d'accueil 3 dans sa position de retenue à la position de déverrouillage dans laquelle il ne verrouille pas cet organe d'accueil et lui permet de passer dans sa position ouverte, ou inversement.

Pour cela, le porte instrument 1 comprend un corps 7 dans lequel l'organe de verrouillage/déverrouillage 4 est monté coulissant, ainsi qu'un ressort de rappel 8 entre le corps 7 et l'organe de verrouillage/déverrouillage 4, le ressort tendant à pousser l'organe de verrouillage/déverrouillage vers la position de verrouillage où il verrouille l'organe d'accueil en position de retenue.

Comme l'illustre la figure 1, l'organe de verrouillage/déverrouillage 4 présente une partie 44, cylindrique, s'étendant hors du corps du porte instrument.

En outre, l'axe 34 permet de solidariser l'organe d'accueil 3 au corps 7, en traversant l'organe de verrouillage/déverrouillage au niveau d'une lumière 42 oblongue (figure 8).

De plus, le corps 7, l'organe d'accueil 3 et l'organe de verrouillage/déverrouillage 4 s'étendent selon la même direction longitudinale. De cette façon, l'organe de verrouillage/déverrouillage 4 est également monté coulissant par rapport à l'organe d'accueil 3.

Le ressort 8 pousse naturellement l'organe de verrouillage/déverrouillage 4 vers sa position de verrouillage.

Dans le sens contraire, une pression de l'étui 5 sur l'organe de verrouillage/déverrouillage comprime le ressort et tend à ramener l'organe de verrouillage/déverrouillage 4 vers sa position de déverrouillage.

### 6.5. Montage et démontage de l'instrument

Lors du montage et du démontage de l'instrument 2 par rapport au porte instrument 1, un équipement pour dentiste selon l'invention présente donc plusieurs configurations, avec des positions qui se suivent dans un ordre prédéfini, conformément à la description suivante.

Cette description est illustrée par les figures 8 à 13, dans lesquelles l'étui représenté est, à titre d'exemple, semi-rigide et tel que décrit précédemment en référence à la figure 5a.

Tout d'abord, la position de verrouillage des moyens de réception et de retenue de l'instrument 2 dans le porte instrument 1, telle qu'on la retrouve lorsque l'équipement pour dentiste est en fonctionnement, est illustrée sur les figures 12 et 13.

La base 31 de l'organe d'accueil 3, en position de retenue, retient fermement l'instrument 2, notamment grâce au serrage de la lèvre intérieure 32 dans la gorge 212. L'organe de verrouillage/déverrouillage 4, qui est dans sa position de verrouillage, maintient l'organe d'accueil 3 en position de retenue.

Cette position de verrouillage est stable, c'est-à-dire que l'instrument 2 reste maintenu dans le porte instrument, sans intervention du praticien, tant qu'aucune autre force extérieure (telle qu'un effort exercé sur l'organe de verrouillage/déverrouillage 4 par l'étui 5) n'est appliquée.

L'étui 5 peut alors être retiré ou inséré par rapport à l'instrument 2 en place, par un effort manuel consistant à faire franchir la lèvre intérieure 52 par rapport à la collerette biseautée 215 (le diamètre de la collerette biseautée étant supérieur à celui de la lèvre intérieure, celle-ci par sa souplesse peut se déformer lors du franchissement).

La figure 13 représente partiellement l'étui, désolidarisé de l'instrument.

Les figures 8 et 9 illustrent une position de déverrouillage des moyens de réception et de retenue, atteinte en poussant l'étui 5 sur l'organe de verrouillage/déverrouillage 4, depuis la position de verrouillage précédente, afin de retirer un instrument 2 du porte instrument 1.

En référence à la figure 8, l'organe de verrouillage/déverrouillage 4 est dans une position déverrouillée, dans laquelle il n'agit pas sur l'organe d'accueil. L'organe d'accueil 3reste cependant dans sa position de retenue, dans laquelle la lèvre intérieure 32 est toujours présente dans la gorge 212, mais de façon non verrouillée, de sorte qu'elle exerce un léger effort de rétention, empêchant l'instrument de chuter gravitairement et permettant au praticien de l'extraire manuellement, par l'intermédiaire de l'étui.

Le retrait de l'instrument monté dans le porte instrument est réalisé grâce à la coopération particulière entre la gorge 214 et la lèvre intérieure 52 de l'étui, permettant de faire passer l'organe d'accueil 3 dans sa position ouverte : dans un sens, d'abord, le praticien force la lèvre intérieure à franchir la collerette biseautée 215 (ou à envelopper l'extrémité cylindrique lisse 2140, dans le cas d'un étui souple), pour chausser l'étui sur l'instrument ; dans le sens contraire, ensuite, une traction sur l'étui force la lèvre intérieure 32 de l'organe d'accueil à s'écarter et à libérer l'instrument, la lèvre intérieure 52 venant alors en butée contre la collerette biseautée 215 (ou, dans le cas d'un étui souple, une pression adaptée des doigts du praticien sur cet étui est suffisante pour saisir l'instrument et le tenir tout au long du retrait).

Pour introduire un nouvel instrument dans le porte instrument, l'organe d'accueil doit également être dans cette position ouverte.

On note que, dans le cas où l'organe de verrouillage/déverrouillage 4 aurait été mis en position de verrouillage alors que l'organe d'accueil ne contient pas d'instrument, une simple pression à l'aide d'un étui vide sur l'organe de verrouillage/déverrouillage 4 suffit à le mettre dans cette position de déverrouillage.

Un instrument 2 peut alors à nouveau être introduit dans l'organe d'accueil. Or, le diamètre extérieur de la queue 21 de l'instrument est supérieur à celui de la lèvre intérieure 32 'au repos'. La lèvre intérieure s'écarte donc progressivement, jusqu'à la position ouverte de l'organe d'accueil, pour laisser pénétrer l'instrument, grâce à la forme arrondie ou chanfreinée de sa queue 21, lorsque le praticien exerce une pression au moyen de l'étui contenant l'instrument. On note que la lèvre intérieure 32 oppose alors une résistance à l'instrument, qui peut amener la lèvre intérieure 52 de l'étui à coulisser jusqu'à la collerette d'arrêt 213.

Dans cette position, l'instrument coulisse dans l'organe d'accueil, jusqu'à ce que l'étui 5 entre en contact avec l'organe de verrouillage/déverrouillage 4. La lèvre intérieure 32 est alors hors de la gorge 212 de l'instrument.

Cette position de déverrouillage de l'organe de verrouillage/déverrouillage est stable, grâce à la présence sur l'organe de verrouillage/déverrouillage d'un ergot 43, élastiquement déformable, coopérant avec un logement 71 réalisé par une découpe dans le corps 7. L'ergot 43 est maintenu en position par l'action conjuguée du ressort 8 et d'une contrainte tangentielle qui le plaque sur le plan 74 du logement 71.

L'équipement reste donc dans cette position, si le praticien relâche la pression mise sur l'étui pour l'atteindre.

Pour atteindre cette position stable de déverrouillage de l'organe de verrouillage/déverrouillage dans le corps, depuis la position de verrouillage, l'ergot change de position mais ne quitte pas le logement 71 (de la position de la figure 13, à la position de la figure 9), en longeant un plan incliné 72, grâce à sa mobilité latérale. Au passage, l'ergot se retrouve calé contre le plan 74, alors qu'il est initialement calé contre un plan 73 parallèle au plan 74.

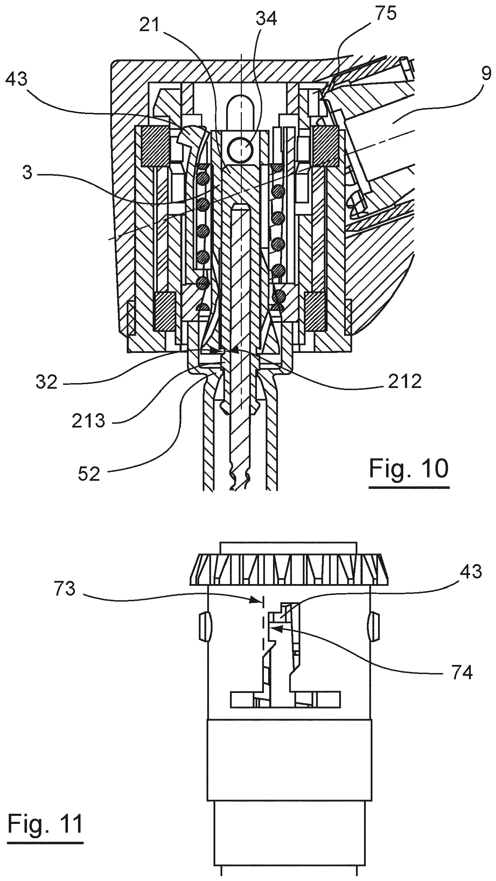

Pour revenir ensuite à la position de verrouillage, permettant de maintenir l'instrument 2 qu'il vient d'introduire dans le porte instrument 1, le dentiste doit d'abord exercer une nouvelle pression de l'étui 5 sur l'organe de verrouillage/déverrouillage 4, jusqu'à ce que ce dernier atteigne la position illustrée par les figures 10 et 11.

Dans cette position, la queue 21 de l'instrument 2 est en butée sur l'axe 34, au fond de l'organe d'accueil 3, la lèvre intérieure 52 de l'étui 5 est au contact de la collerette d'arrêt 213, tandis que la lèvre intérieure 32 de l'organe d'accueil pénètre la gorge 212 de l'instrument.

L'organe de verrouillage/déverrouillage 4 est alors dans une position de déverrouillage, mais qui n'est plus stable. En effet, au cours de la poussée conduisant à cette position, l'ergot 43 reste d'abord dans le logement 71, puis les arêtes chanfreinées et la mobilité radiale de l'ergot lui permettent de sortir du logement 71, selon la figure 11. La position atteinte n'est donc pas stable.

Or, l'ergot 43 sort du logement 71 selon une trajectoire inclinée, par rapport à l'axe de coulissement de l'organe de verrouillage/déverrouillage. Il tend donc à se déplacer latéralement à l'intérieur du corps, hors du logement, en direction du plan 73. Le relâchement progressif de l'étui, conjugué à la poussée du ressort, permet alors à l'organe de verrouillage/déverrouillage de coulisser jusqu'à sa position de verrouillage, sans que l'ergot puisse l'arrêter dans la position de déverrouillage illustrée sur les figures 8 et 9. L'ergot rentre alors à nouveau dans le logement, contre le plan 73. Ainsi, l'organe de verrouillage/déverrouillage 4 présente un ergot 43 mobile radialement et latéralement destiné à coopérer avec un logement 71 ménagé dans le corps 7. Cet egrot permet, avantageusement, de conférer à l'organe de verrouillage/déverrouillage un état bistable, c'est à dire que cet organe de verrouillage/déverrouillage peut être stable aussi bien dans sa position de verrouillage que dans sa position de déverrouillage.

Au cours du mouvement vers sa position de verrouillage, l'organe de verrouillage/déverrouillage 4 comprime la base conique 31 de l'organe d'accueil 3 et la lèvre intérieure 32 reste positionnée dans la gorge 212 de l'instrument 2. A la fin de ce mouvement, l'instrument a donc conservé son positionnement axial par rapport à l'organe d'accueil et est fermement retenu par ce dernier.

L'élasticité de l'organe d'accueil et la présence des évidements longitudinaux permet également d'améliorer le serrage de l'instrument, en augmentant les surfaces en contact avec la queue de l'instrument, de sorte qu'aucun jeu ne subsiste entre l'instrument et l'organe d'accueil.

Une répartition homogène des forces de serrage est en outre obtenue sur toute la surface de la queue de l'instrument. Ainsi, même dans le cas où celle-ci est en plastique, le risque qu'elle se rompe est considérablement réduit, et elle cumule les avantages des matériaux plastiques (facilité d'identification des couleurs, coût réduit...) et des matériaux métalliques (solidité, bonne transmission du couple de rotation...).

En résumé, les moyens de réception et de retenue de l'équipement passe :
- de la position de verrouillage illustrée par la figure 12 à la position de déverrouillage illustrée par la figure 8, lorsque le praticien démonte un instrument présent dans le porte instrument ;
- de la position de déverrouillage illustrée par la figure 8 à la position de verrouillage illustrée par la figure 12, en passant par la position intermédiaire instable illustrée par la figure 10, lorsque le praticien monte un instrument dans le porte instrument.

On note que le corps 7 est, comme l'illustrent les figures 8, 10 et 12, entraîné en rotation grâce à l'engrènement de ses dents 75 avec celles de moyens d'entraînement 9 présents dans le manche du porte instrument 1. Le corps 7 peut ainsi lui-même entraîner l'instrument 2 en rotation.

On note aussi qu'un fonctionnement similaire peut être obtenu en montant un ressort, tel que le ressort 8, entre l'organe de verrouillage/déverrouillage 4 et non pas le corps 7 mais l'organe d'accueil 3. Cette variante peut cependant être moins avantageuse, par le fait que le ressort peut être dans une position gênante lors des changements d'organe d'accueil susceptibles d'être effectués.

### 6.. Moyens de stockage temporaire

Selon un mode de réalisation préférentiel, illustré par la figure 15, l'équipement pour dentiste selon l'invention est utilisé avec un moyen de stockage temporaire 10 pouvant recevoir des étuis 5 destinés à recevoir un instrument 2. Ce moyen est doté d'évidements 101 (trente-deux, en l'occurrence) destinés à recevoir autant d'étuis, d'une façon telle que la partie 51 de chaque étui et la queue 21 de l'instrument éventuellement supporté par l'étui font saillie à l'extérieur du moyen. Ainsi, le praticien peut saisir chaque étui par sa partie 51, ou présenter le porte instrument 1 directement sur la queue 21 de l'instrument.

Ce moyen est particulièrement utile pour supporter, de façon ordonnée, et présenter de façon ergonomique, plusieurs instruments susceptibles d'être utilisés une ou plusieurs fois par le dentiste lors d'une même intervention. Les temps de changements d'instrument sont alors minimisés. Le contact avec l'étui étant réduit, voire supprimé, les conditions aseptiques d'utilisation de l'équipement sont également améliorées.

En particulier, des systèmes autres qu'un ergot élastique peuvent être envisagés pour réaliser les positions stables de verrouillage et de déverrouillage des moyens de réception et de retenue (système rotatif à barillet, notamment).

## Revendications

1. Equipement mécanisé de chirurgie comprenant :
- au moins un porte-instrument (1) comprenant des moyens de réception et de retenue d'un instrument (2) de chirurgie à usage mécanisé, et
- au moins un instrument (2) de chirurgie à usage mécanisé destiné à être monté sur ledit porte-instrument (1), ledit instrument comprenant une queue destinée à coopérer avec le porte instrument et une lame active,
lesdits moyens de réception et de retenue étant mobiles entre :
- une position de déverrouillage, dans laquelle ils permettent l'introduction de ladite queue de l'instrument dans le porte-instrument ou le retrait de ladite queue du porte-instrument, et ;
- une position de verrouillage, dans laquelle elle empêche le retrait de ladite queue du porte instrument ;
et **caractérisé en ce qu'**il comprend un étui (5) dans lequel ladite lame active dudit instrument peut être logée de façon amovible, ledit étui présentant un élément de coopération (51) destiné à coopérer avec lesdits moyens de réception et de retenue pour provoquer leur passage de ladite position de déverrouillage à la position de verrouillage, ou inversement.

2. Equipement mécanisé de chirurgie selon la revendication 1, **caractérisé en ce que** lesdites positions de déverrouillage et de verrouillage des moyens de réception et de retenue sont des positions stables.

3. Equipement mécanisé de chirurgie selon l'une quelconque des revendications précédentes, **caractérisé en ce que** lesdits moyens de réception et de retenue comprennent :
- un organe d'accueil (3) permettant de recevoir et de maintenir ladite queue de l'instrument, ledit organe d'accueil étant mobile entre une position ouverte, autorisant l'introduction de ladite queue dans ledit organe d'accueil ou le retrait de ladite queue dudit organe d'accueil, et une position de retenue dans laquelle elle retient ladite queue dans ledit organe d'accueil ;
- un organe de verrouillage/déverrouillage (4), mobile entre ladite position de verrouillage dans laquelle il maintient ledit organe d'accueil dans ladite position de retenue, et ladite position de déverrouillage dans laquelle il ne maintient pas ledit organe d'accueil dans ladite position de retenue ;
et **en ce que** ledit élément de coopération (51) dudit étuis est conformé pour coopérer avec ledit organe de verrouillage/déverrouillage pour provoquer son passage de ladite position de verrouillage à ladite position de déverrouillage, ou inversement.

4. Equipement mécanisé de chirurgie selon la revendication 3, **caractérisé en ce que** ledit porte-instrument (1) comprend un corps (7) dans lequel l'organe de verrouillage/déverrouillage (4) est monté coulissant, ledit organe de verrouillage/déverrouillage présentant au moins un ergot (43) mobile destiné à coopérer avec au moins un logement (71) ménagé dans ledit corps.

5. Equipement mécanisé de chirurgie selon la revendication 4, **caractérisé en ce que** ledit ergot (43) est mobile radialement et latéralement.

6. Equipement mécanisé de chirurgie selon l'une quelconque des revendications 4 et 5, **caractérisé en ce que** ledit ergot (43) est élastiquement déformable.

7. Equipement mécanisé de chirurgie selon l'une quelconque des revendications 3 à 6, **caractérisé en ce que** ledit organe de verrouillage/déverrouillage (4) est monté coulissant par rapport audit organe d'accueil (3).

8. Equipement mécanisé de chirurgie selon l'une quelconque des revendications 3 à 7, **caractérisé en ce que** ledit organe d'accueil (3) présente une base (31) pourvue d'au moins un doigt (32) mobile radialement, ladite queue de l'instrument (2) présentant au moins un évidement (212) destiné à recevoir ledit doigt dans ladite position de retenue.

9. Equipement mécanisé de chirurgie selon l'une quelconque des revendications 3 à 8, **caractérisé en ce que** ledit porte-instrument (1) comporte un ressort de rappel (8) entre ledit corps (7) et ledit organe de verrouillage/déverrouillage (4), ledit ressort tendant à pousser ledit organe de verrouillage/déverrouillage vers ladite position de verrouillage.

10. Equipement mécanisé de chirurgie selon l'une quelconque des revendications 3 à 9, **caractérisé en ce que** ledit organe de verrouillage/déverrouillage (4) présente une partie (44) s'étendant hors du corps (7) du porte instrument (1).

11. Equipement mécanisé de chirurgie selon la revendication 10, **caractérisé en ce que** ladite partie (44) est cylindrique.

12. Equipement mécanisé de chirurgie selon l'une quelconque des revendications précédente, **caractérisé en ce que** ledit élément de coopération (51) de l'étui (5) est une collerette.

## Patentansprüche

1. Mechanisierte chirurgische Ausrüstung, umfassend:
- mindestens einen Instrumentenhalter (1), der Mittel zum Aufnehmen und Festhalten eines chirurgischen Instrumentes (2) zur mechanisierten Verwendung umfasst, und
- mindestens ein chirurgisches Instrument (2) zur mechanisierten Verwendung, das dazu bestimmt ist, an dem Instrumentenhalter (1) montiert zu werden, wobei das Instrument einen Schaft umfasst, der dazu bestimmt ist, mit dem Instrumentenhalter zusammenzuwirken, und eine aktive Klinge,
wobei die Aufnahme- und Festhaltemittel beweglich sind zwischen:
- einer Entriegelungsstellung, in der sie das Einführen des Schaftes des Instruments in den Instrumentenhalter oder das Herausziehen des Schaftes aus dem Instrumentenhalter ermöglichen; und
- einer Verriegelungsstellung, in der sie das Herausziehen des Schaftes aus dem Instrumentenhalter verhindert;
und **dadurch gekennzeichnet, dass** sie ein Futteral (5) umfasst, in dem die aktive Klinge des Instrumentes herausnehmbar untergebracht werden kann, wobei das Futteral ein Zusammenwirkelement (51) aufweist, das dazu bestimmt ist, mit den Aufnahme- und Festhaltemitteln zusammenzuwirken, um ihren Wechsel von der Entriegelungsstellung in die Verriegelungsstellung oder umgekehrt zu bewirken.

2. Mechanisierte chirurgische Ausrüstung nach Anspruch 1, **dadurch gekennzeichnet, dass** die Entriegelungs- und Verriegelungsstellungen der Aufnahme- und Festhaltemittel stabile Stellungen sind.

3. Mechanisierte chirurgische Ausrüstung nach einem der vorhergehenden Ansprüche, **dadurch gekennzeichnet, dass** die Aufnahme- und Festhaltemittel umfassen:
- ein Empfangsorgan (3), das es ermöglicht, den Schaft des Instrumentes aufzunehmen und zu halten, wobei das Empfangsorgan zwischen einer offenen Stellung, die das Einführen des Schaftes in das Empfangsorgan oder das Herausziehen des Schaftes aus dem Empfangsorgan gestattet, und einer Festhaltestellung beweglich ist, in der sie den Schaft in dem Empfangsorgan festhält;
- ein Verriegelungs-/Entriegelungsorgan (4), das zwischen der Verriegelungsstellung, in der es das Empfangsorgan in der Festhaltestellung hält, und der Entriegelungsstellung beweglich ist, in der es das Empfangsorgan nicht in der Festhaltestellung hält;
und dadurch, dass das Zusammenwirkelement (51) des Futterals dafür ausgebildet ist, mit dem Verriegelungs-/Entriegelungsorgan zusammenzuwirken, um seinen Wechsel von der Verriegelungsstellung in die Entriegelungsstellung oder umgekehrt zu bewirken.

4. Mechanisierte chirurgische Ausrüstung nach Anspruch 3, **dadurch gekennzeichnet, dass** der Instrumentenhalter (1) ein Gehäuse (7) umfasst, in dem das Verriegelungs-/Entriegelungsorgan (4) verschiebbar montiert ist, wobei das Verriegelungs-/Entriegelungsorgan mindestens einen beweglichen Stift (43) aufweist, der dazu bestimmt ist, mit mindestens einer Aufnahme (71), die in dem Gehäuse vorgesehen ist, zusammenzuwirken.

5. Mechanisierte chirurgische Ausrüstung nach Anspruch 4, **dadurch gekennzeichnet, dass** der Stift (43) radial und lateral beweglich ist.

6. Mechanisierte chirurgische Ausrüstung nach einem der Ansprüche 4 und 5, **dadurch gekennzeichnet, dass** der Stift (43) elastisch verformbar ist.

7. Mechanisierte chirurgische Ausrüstung nach einem der Ansprüche 3 bis 6, **dadurch gekennzeichnet, dass** das Verriegelungs-/Entriegelungsorgan (4) in Bezug auf das Empfangsorgan (3) verschiebbar montiert ist.

8. Mechanisierte chirurgische Ausrüstung nach einem der Ansprüche 3 bis 7, **dadurch gekennzeichnet, dass** das Empfangsorgan (3) eine Basis (31) aufweist, die mit mindestens einem radial beweglichen Zapfen (32) versehen ist, wobei der Schaft des Instrumentes (2) mindestens eine Aussparung (212) aufweist, die dazu bestimmt ist, den Zapfen in der Festhaltestellung aufzunehmen.

9. Mechanisierte chirurgische Ausrüstung nach einem der Ansprüche 3 bis 8, **dadurch gekennzeichnet, dass** der Instrumentenhalter (1) eine Rückstellfeder (8) zwischen dem Gehäuse (7) und dem Verriegelungs-/Entriegelungsorgan (4) umfasst, wobei die Feder dazu tendiert, das Verriegelungs-/Entriegelungsorgan in Richtung der Verriegelungsstellung zu drücken.

10. Mechanisierte chirurgische Ausrüstung nach einem der Ansprüche 3 bis 9, **dadurch gekennzeichnet, dass** das Verriegelungs-/Entriegelungsorgan (4) einen Teil (44) aufweist, der sich außerhalb des Gehäuses (7) des Instrumentenhalters (1) erstreckt.

11. Mechanisierte chirurgische Ausrüstung nach Anspruch 10, **dadurch gekennzeichnet, dass** das Teil (44) zylindrisch ist.

12. Mechanisierte chirurgische Ausrüstung nach einem der vorhergehenden Ansprüche, **dadurch gekennzeichnet, dass** das Zusammenwirkelement (51) des Futterals (5) ein Flansch ist.

## Claims

1. Mechanised surgical equipment comprising:
- at least one instrument holder (1) comprising means for receiving and holding a surgical instrument (2) for mechanised use, and
- at least one surgical instrument (2) for mechanised use intended to be mounted on said instrument holder (1), said instrument comprising a shank intended to engage with the instrument holder and an active blade,
said receiving and holding means being movable between:
- an unlocking position, wherein they allow the insertion of said shank of the instrument into the instrument holder or the removal of said shank from the instrument holder, and;
- a locking position, wherein it prevents the removal of said shank from the instrument holder;
and **characterised in that** it comprises a sheath (5) wherein said active blade of said instrument may be removably housed, said sheath having an engagement element (51) intended to engage with said receiving and holding means to induce the switch thereof from said unlocking position to the locking position, or vice versa.

2. Mechanised surgical equipment according to claim 1, **characterised in that** said unlocking and locking positions of the receiving and holding means are stable positions.

3. Mechanised surgical equipment according to any one of the preceding claims, **characterised in that** said receiving and holding means comprise:
- a receiving member (3) for receiving and holding said shank of the instrument, said receiving member being movable between an open position, allowing the insertion of said shank into said receiving member or the removal of said shank from said receiving member, and a holding position wherein it holds said shank in said receiving member;
- a locking/unlocking member (4), movable between said locking position wherein it holds said receiving member in said holding position, and said unlocking position wherein it does not hold said receiving member in said holding position;
and **in that** said engagement element (51) of said sheath is formed to engage with said locking/unlocking member to induce the change thereof from said locking position to said unlocking position, or vice versa.

4. Mechanised surgical equipment according to claim 3, **characterised in that** said instrument holder (1) comprises a body (7) wherein the locking/unlocking member (4) is slidably mounted, said locking/unlocking member having at least one movable lug (43) intended to engage with at least one housing (71) formed in said body.

5. Mechanised surgical equipment according to claim 4, **characterised in that** said lug (43) is radially and laterally movable.

6. Mechanised surgical equipment according to any one of claims 4 and 5, **characterised in that** said lug (43) is elastically deformable.

7. Mechanised surgical equipment according to any one of claims 3 to 6, **characterised in that** said locking/unlocking member (4) is slidably mounted relative to said receiving member (3).

8. Mechanised surgical equipment according to any one of claims 3 to 7, **characterised in that** said receiving member (3) has a base (31) provided with at least one radially movable finger (32), said shank of the instrument (2) having at least one recess (212) intended to receive said finger in said holding position.

9. Mechanised surgical equipment according to any one of claims 3 to 8, **characterised in that** said instrument holder (1) includes a return spring (8) between said body (7) and said locking/unlocking member (4), said spring tending to push said locking/unlocking member towards said locking position.

10. Mechanised surgical equipment according to any one of claims 3 to 9, **characterised in that** said locking/unlocking member (4) has a portion (44) extending outside of the body (7) of the instrument holder (1).

11. Mechanised surgical equipment according to claim 10, **characterised in that** said portion (44) is cylindrical.

12. Mechanised surgical according to any one of the preceding claims, **characterised in that** said engagement element (51) of the sheath (5) is a collar.
